(19) Europäisches Patentamt
European Patent Office
Office européen des brevets

(11) Veröffentlichungsnummer: **0 058 896 A1**

(12) **EUROPÄISCHE PATENTANMELDUNG**

(21) Anmeldenummer: **82101054.3**

(22) Anmeldetag: **12.02.82**

(51) Int. Cl.³: **C 07 C 118/02**, C 07 C 119/042

(30) Priorität: **24.02.81 DE 3106774**

(43) Veröffentlichungstag der Anmeldung: **01.09.82 Patentblatt 82/35**

(84) Benannte Vertragsstaaten: **BE DE FR GB IT**

(71) Anmelder: **BAYER AG, Zentralbereich Patente, Marken und Lizenzen, D-5090 Leverkusen 1, Bayerwerk (DE)**

(72) Erfinder: **Buschhaus, Hans-Ulrich, Dr., Morgengraben 2, D-5000 Köln 80 (DE)**
Erfinder: **Findeisen, Kurt, Dr., In der Follmühle 10, D-5068 Odenthal 2 (DE)**
Erfinder: **Burkhardt, Tilo, Dr., Höffenstrasse 16, D-5060 Bergisch Gladbach 2 (DE)**

(54) **Verfahren zur Herstellung von Isocyanatocarbonsäureestern.**

(57) Die Erfindung betrifft ein neues Verfahren zur Herstellung von Isocyanatocarbonsäureestern in einem Eintopf-Verfahren, bei welchem man Aminocarbonsäuren in Gegenwart von Chlorwasserstoff mit Alkoholen zu Aminocarbonsäureester-Hydrochloriden umsetzt und diese anschliessend, gegebenenfalls nach destillativer Entfernung des überschüssigen Alkohols, in Gegenwart von flüssigen Säurechloriden als Lösungs- bzw. Suspendiermittel phosgeniert.

EP 0 058 896 A1

0058896

BAYER AKTIENGESELLSCHAFT
Zentralbereich
Patente, Marken und Lizenzen
5090 Leverkusen, Bayerwerk
Wr/bc/c

# Verfahren zur Herstellung von Isocyanatocarbonsäureestern

Die Erfindung betrifft ein neuartiges Verfahren zur Herstellung von Isocyanatocarbonsäureestern, bei welchem man Aminocarbonsäuren in einem ersten Reaktionsschritt in Gegenwart von Chlorwasserstoff mit Alkoholen zu Aminocarbonsäureester-Hydrochloriden umsetzt und diese anschließend, gegebenenfalls nach destillativer Entfernung von überschüssigem Alkohol, in einem Eintopf-Verfahren in Gegenwart von flüssigen Säurechloriden phosgeniert.

Isocyanatocarbonsäureester sind an sich bekannt (DE-OS 1 543 243, W. Siefken, Ann. 562, 105 (1949), (O. Diels, B. Wolff), Ber. 39, 686 (1906); DE-PS 1 085 869). Wie diesen Vorveröffentlichungen entnommen werden kann, müssen bei den Verfahren des Standes der Technik zunächst in völlig getrennten Verfahren aus Aminosäuren die Aminosäureestersalze hergestellt und von Wasser- und Alkoholresten befreit werden, bevor sie in inerten Lösungsmitteln phosgeniert werden können.

Nachteilig ist hierbei die aufwendige und ausbeutemindernde Isolierung und Trocknung der Aminosäureestersalze. Ein weiterer Nachteil der erwähnten Verfahren besteht darin, daß die Umsetzung der Aminosäureestersalze mit Phosgen teilweise oder ganz bei Temperaturen von 140 bis 200°C durchgeführt werden muß, da die Dehydrohalogenierung der Carbaminsäurechloride und die Reaktion zwischen Aminosäureesterhydrochloriden und Phosgen nur bei einer so hohen Temperatur abläuft; solche Temperaturen können jedoch zu einer Esterspaltung und einer damit verbundenen Ausbeuteminderung führen.

Gemäß DE-OS 1 543 243 ist es zur Erzielung guter Ausbeuten erforderlich, gewisse Aminosäureestersalze vor der Phosgenierungsreaktion feinteilig zu zermahlen, was zu einer Verteuerung der Reaktionsdurchführung führt.

Es war daher die Aufgabe der vorliegenden Erfindung, ein verbessertes Verfahren zur Herstellung von Isocyanatocarbonsäureestern zur Verfügung zu stellen, welches nicht mit den Nachteilen der Verfahren des Standes der Technik behaftet ist. Diese Aufgabe konnte überraschenderweise durch das nachstehend näher beschriebene erfindungsgemäße Verfahren gelöst werden.

Gegenstand der Erfindung ist ein Verfahren zur Herstellung von Isocyanatocarbonsäureestern durch Phosgenierung von Aminocarbonsäureester-Hydrochloriden,

die durch Veresterung von Aminocarbonsäuren mit Alkoholen in Gegenwart von Chlorwasserstoff erhalten worden sind, in Gegenwart von Lösungsmitteln, dadurch gekennzeichnet, daß man

a) die durch Veresterung von Aminocarbonsäuren mit Alkoholen in Gegenwart von Chlorwasserstoff erhaltenen Aminocarbonsäureester-Hydrochloride, gegebenenfalls nach destillativer Entfernung von überschüssigem Alkohol, ohne weitere Aufarbeitung in einem Eintopf-Verfahren phosgeniert und

b) die Phosgenierungsreaktion in Gegenwart von unter den Reaktionsbedingungen flüssigen und gegenüber Wasser und alkoholischen Hydroxylgruppen reaktionsfähigen Säurechloriden als Lösungs- bzw. Suspendiermittel durchführt.

Aus der DE-OS 2 329 051 war zwar bereits bekannt, daß Phosphoroxichlorid ein zur Phosgenierung von primären Aminen geeignetes Lösungsmittel darstellt. Dieser Vorveröffentlichung ist jedoch keinerlei Anregung zu entnehmen, zur Lösung der ganz spezifischen, mit der Herstellung von Isocyanatocarbonsäureestern in Zusammenhang stehenden Probleme ein solches oder ähnliches Lösungsmittel einzusetzen.

Beim erfindungsgemäßen Verfahren können beliebige, ausschließlich primäre Aminogruppen aufweisende, von den Amino- und Carbonsäuregruppen abgesehen,

unter den Reaktionsbedingungen inerte Aminocarbonsäuren eingesetzt werden. Besonders gut geeignete Aminocarbonsäuren sind beispielsweise solche der Formel

$$(HOOC)_m\text{-}R\text{-}(NH_2)_n$$

in welcher

m und n jeweils für ganze Zahlen von 1 bis 3 stehen und

R für einen (m+n)-wertigen aliphatischen Kohlenwasserstoffrest mit 1 bis 18, vorzugsweise 1 bis 7, Kohlenstoffatomen, cycloaliphatischen Kohlenwasserstoffrest mit 4 bis 20, vorzugsweise 6 bis 10, Kohlenstoffatomen, cycloaliphatisch-aliphatischen Kohlenwasserstoffrest mit insgesamt 4 bis 20, vorzugsweise 6 bis 10, Kohlenstoffatomen, araliphatischen Kohlenwasserstoffrest mit 7 bis 15, vorzugsweise 7 bis 10, Kohlenstoffatomen oder einen aromatischen Kohlenwasserstoffrest mit 6 bis 20, vorzugsweise 6 bis 10 Kohlenstoffatomen steht, wobei der Rest R jeweils inerte Substituenten, wie insbesondere Halogenatome aufweisen oder durch Brückenglieder wie -O-, -S- oder $-SO_2-$ unterbrochen sein kann.

Besonders gut geeignete Aminocarbonsäuren sind solche der genannten allgemeinen Formel, für welche

m für 1 steht,
n für 1 oder 2, insbesondere für 2 steht und
R für einen gesättigten aliphatischen Kohlenwasserstoffrest mit 1 bis 7 Kohlenstoffatomen steht.

Allen für das erfindungsgemäße Verfahren geeigneten Aminocarbonsäuren der genannten allgemeinen Formel gemeinsam ist der Umstand, daß bei Vorliegen von mehreren Aminogruppen (n = 2 oder 3) diese mit verschiedenen Kohlenstoffatomen des Restes R verknüpft sind.

Typische Vertreter von für das erfindungsgemäße Verfahren geeigneten Aminocarbonsäuren sind z.B. Aminoessigsäure, 2-Aminopropansäure, 3-Aminopropansäure, 3-Aminocapronsäure, 4-Aminocapronsäure, 5-Aminocapronsäure, 6-Aminocapronsäure, 2,6-Diaminocapronsäure (Lysin), 3,6-Diaminocapronsäure, p-Aminobenzoesäure, m-Aminobenzoesäure, o-Aminobenzoesäure, Arginin, Asparagin, Asparaginsäure, Glutaminsäure, Glutamin, Isoleucin, Leucin, Methionin, Phenylalanin, Threonin, Valin. 2,6-Diaminocapronsäure (Lysin) ist besonders bevorzugt.

Für das erfindungsgemäße Verfahren geeignete Alkohole sind beliebige organische Verbindungen, die mindestens eine alkoholische Hydroxylgruppe und gegebenenfalls eine oder mehrere primäre Aminogruppen aufweisen und ansonsten unter den Bedingungen des erfindungsgemäßen Verfahrens inert sind. Typische Vertreter geeigneter Alkohole sind beispielsweise

solche der allgemeinen Formel

$$(H_2N)_o-R'-(OH)_p$$

in welcher

o für 0 oder 1, vorzugsweise 0 steht,

p für eine ganze Zahl von 1 bis 4, vorzugsweise 1 steht und

R' für einen (o+p)-wertigen aliphatischen Kohlenwasserstoffrest mit 1 bis 18, vorzugsweise 1 bis 4, Kohlenstoffatomen, cycloaliphatischen Kohlenwasserstoffrest mit 4 bis 18, vorzugsweise 6 bis 10, Kohlenstoffatomen oder araliphatischen Kohlenwasserstoffrest mit 7 bis 15, vorzugsweise 7 bis 10, Kohlenstoffatomen steht, wobei der Rest R' inerte Substituenten oder Brückenglieder der bei der Definition von R beispielhaft genannten Art aufweisen kann.

Besonders gut geeignete Alkohole sind solche der genannten allgemeinen Formel, für welche

o für 0 steht,

p für 1 steht und

R' für einen gesättigten aliphatischen Kohlenwasserstoffrest mit 1 bis 4 Kohlenstoffatomen steht.

Allen Alkoholen der genannten allgemeinen Formel gemeinsam ist der Umstand, daß die Amino- und Hydroxylgruppen jeweils an verschiedenen Kohlenstoffatomen des Restes R angeordnet sind. Im Falle der Verwendung von mehrwertigen Alkoholen (p = 2, 3 oder 4) werden beim erfindungsgemäßen Verfahren einbasische Carbonsäuren (m = 1) eingesetzt. Die Verwendung von Aminoalkoholen und/oder von mehrwertigen Alkoholen führt zu einer gegebenenfalls erwünschten Erhöhung der NCO-Funktionalität in den erfindungsgemäßen Verfahrensprodukten.

Typische Vertreter von für das erfindungsgemäße Verfahren geeigneten Alkoholen sind z.B. Methanol, Ethanol, n-Propanol, Isopropanol, n-Butanol, 2-Aminoethanol, 3-Aminopropanol, 2-Aminopropanol, Ethylenglykol, Butandiol-1,4, Butandiol-2,3, Trimethylolpropan, Pentaerythrit oder Cyclohexanol. Vorzugsweise werden beim erfindungsgemäßen Verfahren unter den Reaktionsbedingungen flüssige Alkohole der beispielhaft genannten Art eingesetzt. Die beispielhaft genannten einfachen Alkohole wie Methanol, Ethanol, m-Propanol, Isopropanol oder n-Butanol sind besonders gut geeignet. Selbstverständlich können auch beliebige Alkoholgemische eingesetzt werden.

Bei der Durchführung der ersten Stufe des erfindungsgemäßen Verfahrens werden mindestens solche Mengen des Alkohols eingesetzt, daß auf jede Carboxylgruppe der Aminocarbonsäure eine Hydroxylgruppe des Alkohols entfällt. Bei der weniger bevorzugten Ausführungsform des erfindungsgemäßen Verfahrens, gemäß welcher schwerflüchtige, destillativ nicht entfernbare Alkohole eingesetzt werden, wird vorzugsweise unter Verwendung

äquivalenter Mengen der Reaktionspartner gearbeitet. In einem solchen Falle empfiehlt sich die Mitverwendung von inerten Lösungsmitteln wie z.B. aliphatische, cycloaliphatische und/oder aromatische Kohlenwasserstoffe oder halogenierte Kohlenwasserstoffe.

Bei der bevorzugten Ausführungsform des erfindungsgemäßen Verfahrens, gemäß welcher leicht flüchtige einfache Alkohole der beispielhaft genannten Art eingesetzt werden, wird vorzugsweise ein Überschuß des Alkohols eingesetzt, der gleichzeitig als Reaktionsmedium dient. Gemäß dieser bevorzugten Variante werden im allgemeinen solche Mengen an Alkohol eingesetzt, daß bei der Veresterungsreaktion 5- bis 50-gew.-%ige, vorzugsweise 10- bis 20-gew.-%ige Lösungen bzw. Suspensionen der Aminocarbonsäureester-Hydrochloride im überschüssigen Alkohol vorliegen.

Die erste Stufe des erfindungsgemäßen Verfahrens erfolgt in Gegenwart von Chlorwasserstoff. Dies bedeutet, daß entweder vorab hergestellte Hydrochloride der Aminocarbonsäuren eingesetzt werden, und/oder daß man dem Reaktionsgemisch aus Aminocarbonsäure und Alkohol Chlorwasserstoff beispielsweise in Form eines Chlorwasserstoff-Gasstromes einverleibt. Die Menge des Chlorwasserstoffs wird zumindest so bemessen, daß alle Aminogruppen in Ammoniumgruppen überführt werden. Bei Verwendung eines Chlorwasserstoff-Gasstroms kann selbstverständlich ein beliebig großer Chlorwasserstoffüberschuß zur Anwendung gelangen.

Die erste Stufe des erfindungsgemäßen Verfahrens erfolgt im allgemeinen im Temperaturbereich von 20 bis 120, vorzugsweise 60 bis 80 °C.

Nach Beendigung der ersten Stufe des erfindungsgemäßen Verfahrens wird der gegebenenfalls im Überschuß vorliegende flüchtige Alkohol destillativ entfernt. Das gemäß der weniger bevorzugten Ausführungsform gegebenenfalls mitverwendete Hilfslösungsmittel wird ebenfalls vorzugsweise vor der Durchführung der zweiten Stufe des erfindungsgemäßen Verfahrens destillativ abgezogen. Das so erhaltene Reaktionsgemisch kann dann ohne weitere Aufarbeitung in einem Eintopf-Verfahren der zweiten Stufe des erfindungsgemäßen Verfahrens zugeführt werden.

Die zweite Stufe des erfindungsgemäßen Verfahrens, die Phosgenierungsreaktion, wird in Gegenwart von Säurechloriden, die unter den Bedingungen der Phosgenierungsreaktion gegenüber Wasser und gegenüber alkoholischen Hydroxylgruppen reaktionsfähig sind, als Lösungs- bzw. Suspendiermittel durchgeführt. Die Säurechloride stellen im allgemeinen für die Aminocarbonsäureester-Hydrochloride ein Suspendiermittel und für die erfindungsgemäßen Verfahrensprodukte ein Lösungsmittel dar. Selbstverständlich ist es jedoch auch möglich, beim erfindungsgemäßen Verfahren solche Aminocarbonsäureester-Hydrochloride bzw. solche Säurechloride einzusetzen, die sich zu klaren Lösungen vereinigen lassen.

Beispiele geeigneter Säurechloride sind Acetylchlorid, Propionylchlorid, Phosphortrichlorid, Phosphoroxichlorid, Sulfonylchlorid oder Sulfurylchlorid. Besonders bevorzugt wird Phosphoroxichlorid verwendet.

Die Säurechloride werden im allgemeinen in solchen Mengen verwendet, daß 5- bis 40-gew.-%ige, vorzugsweise 5- bis 15-gew.-%ige Suspensionen bzw. Lösungen der Aminocarbonsäureester-Hydrochloride vorliegen.

In die so erhaltenen Suspensionen bzw. Lösungen wird zur Durchführung der zweiten Stufe des erfindungsgemäßen Verfahrens beispielsweise unter innigem Durchmischen Phosgen eingeleitet. Dies erfolgt im allgemeinen bei einer Anfangstemperatur von etwa -10°C bis etwa 40°C und insbesondere von 20 bis 30°C und einer Endtemperatur von etwa 40 bis etwa 100°C, vorzugsweise etwa 60 bis 90°C. Diese Art der Temperaturführung ist jedoch für die Durchführung der zweiten Stufe des erfindungsgemäßen Verfahrens nicht wesentlich. So ist es durchaus auch möglich, die Gesamtreaktion beispielsweise bei 40 bis 100°C durchzuführen. Die Phosgeneinleitung wird bis zur beispielsweise durch IR-Spektralanalyse erkennbaren Beendigung der Reaktion aufrechterhalten. Die Phosgenierungsreaktion kann sowohl unter Normalals auch unter Überdruck durchgeführt werden. Nach Beendigung der Umsetzung wird das Reaktionsgemisch destillativ aufgearbeitet.

Das erfindungsgemäße Verfahren ermöglicht die Herstellung von Isocyanatocarbonsäureestern, ausgehend von Aminosäuren in einem Eintopf-Verfahren. Eine

umständliche Reindarstellung der als Zwischenprodukte anfallenden Aminocarbonsäureester-Hydrochloride ist hierbei nicht erforderlich. Als weitere Vorteile des erfindungsgemäßen Verfahrens gegenüber den Verfahren des Standes der Technik sind die sehr guten Ausbeuten, vergleichsweise niedrigen Reaktionstemperaturen bei der Phosgenierung und die kurzen Phosgenierzeiten hervorzuheben. Eine feinteilige Zermahlung der Aminocarbonsäureester-Hydrochloride ist beim erfindungsgemäßen Verfahren im Gegensatz zum Verfahren gemäß DE-OS 1 543 243 nicht erforderlich.

Beim erfindungsgemäßen Verfahren entstehen als Verfahrensprodukte im Falle der Verwendung von Aminocarbonsäuren der o.g. allgemeinen Formel, für welche m und n für 1 stehen und im Falle der gleichzeitigen Verwendung von Aminogruppen-freien, einwertigen Alkoholen Carbonsäureestergruppen aufweisende Monoisocyanate, die wertvolle Zwischenprodukte zur Herstellung von Schädlingsbekämpfungsmitteln darstellen. Im Falle der Verwendung von Polyaminocarbonsäuren der genannten allgemeinen Formel, für welche n für 2 oder 3 steht und/oder im Falle der Verwendung von mehrwertigen Alkoholen der obengenannten allgemeinen Formel, für welche o für 0 und p für eine ganze Zahl von 2 bis 4 steht und/oder im Falle der Verwendung von Aminoalkoholen der oben beispielhaft genannten Art entstehen beim erfindungsgemäßen Verfahren Carbonsäureestergruppen aufweisende Polyisocyanate, die wertvolle Ausgangsmaterialien für die Herstellung von Polyurethankunststoffen nach dem Isocyanat-Polyadditionsverfahren darstellen.

Die nachfolgenden Beispiele dienen zur weiteren Erläuterung des erfindungsgemäßen Verfahrens. Alle Prozentangaben beziehen sich auf Gewichtsprozente.

Beispiel 1

Isocyanatoessigsäuremethylester

7,5 g (0,1 Mol) Aminoessigsäure werden in 50 ml Methanol suspendiert und trockenes HCl-Gas ohne Kühlung eingeleitet. Nach Bildung einer klaren Lösung wird noch 1 Stunde bei 65°C HCl-Gas eingeleitet. Das Methanol wird zunächst bei Normaldruck, dann bei vermindertem Druck weitgehend abdestilliert und der farblose , kristalline Rückstand in 100 ml Phosphoroxichlorid suspendiert.

In die Suspension wird, beginnend bei Raumtemperatur, Phosgen eingeleitet. Unter weiterer Phosgenzufuhr wird auf 95°C aufgeheizt. Nach 1 Stunde Phosgeneinleiten entsteht eine klare, farblose Lösung. Die Phosgenzufuhr wird beendet, überschüssiges Phosgen mit Stickstoff ausgeblasen und der Ansatz destilliert.
Es werden so 10,7 g (93,2 % der Theorie) Isocyanatoessigsäuremethylester als bei 76°C/32 mbar siedende Fraktion erhalten.

Beispiel 2

Isocyanatoessigsäureethylester

7,5 g (0,1 Mol) Aminoessigsäure werden mit 50 ml Ethanol, wie in Beispiel 1 beschrieben, verestert. Nach der Veresterung entfernt man destillativ das Ethanol, suspendiert den Rückstand in 100 ml Phosphoroxichlorid und

leitet bei 25°C Phosgen ein. Man erhöht die Temperatur auf 90°C und phosgeniert 1 Stunde unter Bildung einer farblosen, klaren Lösung. Überschüssiges Phosgen wird mit Stickstoff ausgeblasen und der Ansatz destilliert. Es werden 12,1 g (94,1 % der Theorie) Isocyanatoessigsäureethylester als bei 80°C/27 mbar siedende Fraktion erhalten.

Beispiel 3

2,6-Diisocyanatohexancarbonsäuremethylester

91,5 g (0,5 Mol) 2,6-Diaminohexancarbonsäuremonohydrochlorid werden in 500 ml Methanol suspendiert und ohne Kühlen mit HCl-Gas gesättigt. Es entsteht eine klare, farblose Lösung aus der der Ester bei weiterem HCl-Einleiten bei 65°C auskristallisiert. Das Methanol wird weitgehend abdestilliert. Der Rückstand wird in 750 ml Phosphoroxichlorid suspendiert und unter Phosgen-Einleitung auf 95°C erhitzt. Nach 6 Stunden Phosgenierung ist der Ester unter Bildung einer fast farblosen, klaren Lösung umgesetzt. Überschüssiges Phosgen wird mit Stickstoff ausgeblasen. Der Ansatz wird destilliert. Es werden 96,1 g (90,7 % der Theorie) 2,6-Diisocyanatohexancarbonsäuremethylester als bei 110 bis 112°C/0,1333 mbar siedende Fraktion erhalten.

Beispiel 4

6-Isocyanatohexancarbonsäuremethylester

13,1 g (0,1 Mol) 6-Aminocapronsäure werden in 50 ml Methanol suspendiert und HCl-Gas bis zur Sättigung ohne Kühlung eingeleitet. Die HCl-Einleitung wird bei 65°C 3 Stunden beibehalten. Anschließend destilliert man das Methanol weitgehend ab.

Der farblose, kristalline Rückstand wird in 100 ml Phosphoroxichlorid suspendiert und Phosgen eingeleitet. Man erhitzt auf 95°C, leitet 1 Stunde Phosgen unter Bildung einer klaren Lösung ein und bläst überschüssiges Phosgen mit Stickstoff aus. Es werden 14,9 g 6-Isocyanatohexancarbonsäuremethylester (87,3 % der Theorie) als bei 82°C/0,05 mbar siedende Fraktion erhalten.

Beispiel 5

6-Isocyanatohexancarbonsäureethylester

13,1 g (0,1 Mol) 6-Aminocapronsäure werden in 100 ml Ethanol suspendiert und HCl-Gas ohne Kühlen bis zur Sättigung eingeleitet. Die HCl-Einleitung wird bei 80°C 3 Stunden fortgeführt.

Man destilliert das Ethanol weitgehend aus, nimmt den Rückstand in 100 ml Phosphoroxichlorid auf, leitet

bei 25°C Phosgen ein und erwärmt während des Einleitens auf 95°C. Nach 90 Minuten vertreibt man mit Stickstoff überschüssiges Phosgen und destilliert. Es werden 16,6 g 6-Isocyanatohexancarbonsäureethylester (89,7 % der Theorie) als bei 84°C/0,1333 mbar siedende Fraktion erhalten.

Vergleichsbeispiel

116,5 g (0,5 Mol) 2,6-Diaminohexansäuremethylesterdihydrochlorid, dargestellt wie in Beispiel 3, werden 12 Stunden bei 80°C/15 Torr getrocknet.

250 g Phosgen werden in 750 ml o-Dichlorbenzol bei 0°C im vorgenannten Lösungsmittel verflüssigt. Man gibt den getrockneten Ester bei 0°C hinzu und erwärmt innerhalb 5 Stunden auf 150°C, wobei weiter Phosgen eingeleitet wird. Dann wird 16 Stunden bei 165°C phosgeniert.

Überschüssiges Phosgen wird mit Stickstoff ausgeblasen, von ungelösten Stoffen filtriert und destilliert. Es werden 17,7 g 2,6-Diisocyanatohexancarbonsäuremethylester (16,7 % der Theorie) als bei 110 bis 112°C/0,1333 mbar siedende Fraktion erhalten.

Patentansprüche

1) Verfahren zur Herstellung von Isocyanatocarbonsäureestern durch Phosgenierung von Aminocarbonsäureester-Hydrochloriden, die durch Veresterung von Aminocarbonsäuren mit Alkoholen in Gegenwart von Chlorwasserstoff erhalten worden sind, in Gegenwart von Lösungsmitteln, dadurch gekennzeichnet, daß man

a) die durch Veresterung von Aminocarbonsäuren mit Alkoholen in Gegenwart von Chlorwasserstoff erhaltenen Aminocarbonsäureester-Hydrochloride, gegebenenfalls nach destillativer Entfernung von überschüssigem Alkohol, ohne weitere Aufarbeitung in einem Eintopf-Verfahren phosgeniert und

b) die Phosgenierungsreaktion in Gegenwart von unter den Reaktionsbedingungen flüssigen und gegenüber Wasser und alkoholischen Hydroxylgruppen reaktionsfähigen Säurechloriden als Lösungs- bzw. Suspendiermittel durchführt.

2) Verfahren gemäß Anspruch 1, dadurch gekennzeichnet, daß man als Säurechlorid Phosphoroxichlorid verwendet.

3) Verfahren gemäß Ansprüchen 1 und 2, dadurch gekennzeichnet, daß man als Aminosäure Lysin verwendet.

4) Verfahren gemäß Ansprüchen 1 bis 3, dadurch gekennzeichnet, daß man als Alkohol ein einwertiges Alkanol mit 1 bis 4 Kohlenstoffatomen verwendet.

0058896

Europäisches Patentamt

# EUROPÄISCHER RECHERCHENBERICHT

Nummer der Anmeldung

EP 82 10 1054

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| D/Y | DE - A - 2 329 051 (BAYER A.G.)<br>* Seite 11 *<br>-- | 1-4 | C 07 C 118/02<br>119/042 |
| Y | FR - A - 1 507 037 (ROHM AND HAAS)<br>* Seite 3 *<br>& DE - A - 1 593 962<br>------- | 1-4 | |
| | | | RECHERCHIERTE SACHGEBIETE (Int. Cl.3) |
| | | | C 07 C 118/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 19-05-1982 | VERHULST |

EPA form 1503.1 06.78